# EUROPEAN PATENT APPLICATION

(11) **EP 3 192 474 A1**
(43) Date of publication of application: **19.07.2017**
(21) Application number: 15840049.9
(22) Date of filing: 18.08.2015
(51) Int. Cl.: A61F 2/86, A61F 2/88, A61L 27/50

(54) **STENT AND METHOD FOR MANUFACTURING STENT**

(30) Priority: 12.09.2014 KR 20140121345
(71) Applicant: CG Bio Co., Ltd., Jungwon-gu Seongnam-si, Gyeonggi-do 462-807 (KR)
(72) Inventor: KIM, Seong Hyeon, Yongin-si Gyeonggi-do 16892 (KR); KI, Byoung Yun, Seongnam-si Gyeonggi-do 13500 (KR)
(74) Representative: Hoppe, Georg Johannes
(86) International application number: PCT/KR2015/008584
(87) International publication number: WO 2016/039533

(57) **Abstract**

Disclosed herein is a stent.

The stent includes: a stent body in which a plurality of band parts having circumferences formed by supporting parts arranged in a connection state having a zigzag shape are formed in a connection state in which they configure a cylindrical shape; and a spiral support part extended and formed in a spiral wound state from one end portion of the stent body in a length direction toward the other end portion of the stent body in the length direction in a state in which it configures a supporting part of any one point among supporting parts of the respective band parts.

## Description

### [Technical Field]

The present invention relates to a stent and a method of manufacturing the same. More particularly, the present invention relates to a stent capable of stably providing elastic restoring force, and a method of manufacturing the same.

### [Background Art]

In general, there are several types of stents having structures matched to stenting parts of a human body or demanded functions depending on the stenting parts or the demanded functions.

Particularly, as a stent having a structure with elastic restoring force, there is a stent having a self expansion type structure formed by banding a wire so that a circumference part of the stent has self elastic force, and it is important to form this stent so as to have a wire entanglement structure in which the circumference part may maintain its original form by the elastic force.

As the stent having the self expansion type structure, there is a stent as disclosed in Korean Patent No. 10-0974308.

However, according to a wire entanglement structure disclosed in Korean Patent No. 10-0974308, an entanglement structure (for example, a support) for securing stable elastic restoring force is not provided, such that it is difficult to expect satisfactory stenting stability and stenting reliability at the time of performing stenting.

### [DISCLOSURE]

### [Technical Problem]

The present invention has been made in an effort to provide a stent and a method of manufacturing the same having advantages of securing stable elastic restoring force by a simple support structure.

### [Technical Solution]

An exemplary embodiment of the present invention provides a stent including: a stent body in which a plurality of band parts having circumferences formed by supporting parts arranged in a connection state having a zigzag shape are formed in a connection state in which they configure a cylindrical shape; and a spiral support part extended and formed in a spiral wound state from one end portion of the stent body in a length direction toward the other end portion of the stent body in the length direction in a state in which it configures a supporting part of any one point among supporting parts of the respective band parts.

Another exemplary embodiment of the present invention provides a method of manufacturing a stent, including: forming a spiral support part by banding a wire so as to have a spiral wound state from one side toward the other side; and banding the wire in a state in which the spiral support part configures a portion of a circumference to form a plurality of band parts and forming a stent body so that the band parts configure a connection state having a cylindrical shape.

### [Advantageous Effects]

As described above, the stent according to an exemplary embodiment of the present invention may include the spiral support part to secure elastic restoring force of a circumference of the stent body.

Particularly, the spiral support part, which is a simple support structure configuring a supporting part (some of circumferences) of any one point among circumferences of the respective band parts configuring the stent body, may provide uniform and stable restoring force to the entire circumference of the stent body.

### [Description of the Drawings]

FIG. 1 is a view schematically showing an entire structure of a stent according to an exemplary embodiment of the present invention.
FIG. 2 is a view showing processes of a method of manufacturing a stent according to an exemplary embodiment of the present invention.
FIGS. 3 to 12 are views for describing the respective processes of FIG. 2.

### [Mode for Invention]

Hereinafter, exemplary embodiments of the present invention will be described with reference to the accompanying drawings.

Exemplary embodiments of the present invention will be described so that those skilled in the art may execute the present invention.

Therefore, exemplary embodiments of the present invention may be modified into several other forms. Accordingly, the claims of the present invention are not limited to exemplary embodiments to be described below.

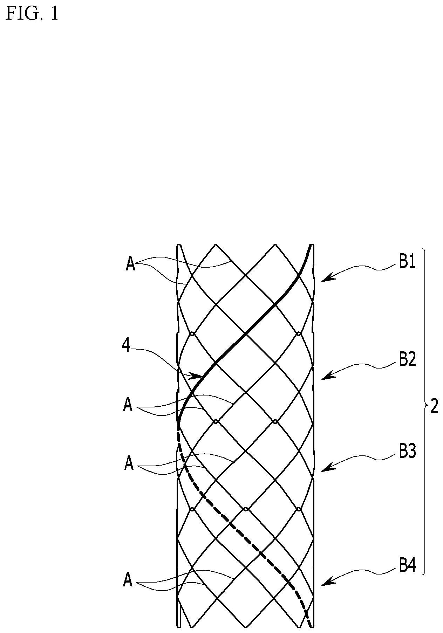

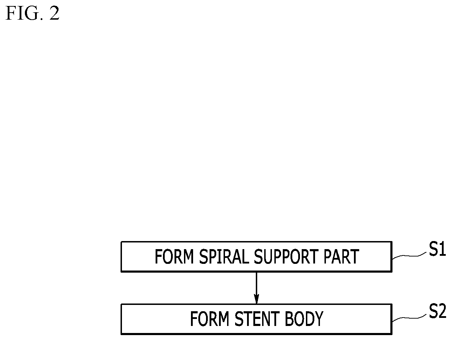

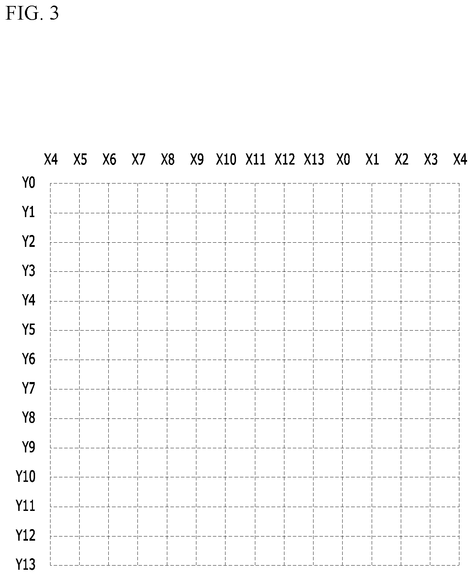

FIG. 1 is a view schematically showing an entire structure of a stent according to an exemplary embodiment of the present invention, FIG. 2 is a view showing processes of a method of manufacturing a stent according to an exemplary embodiment of the present invention, and FIGS. 3 to 12 are views for describing the respective processes of FIG. 2.

Referring to FIG. 1, the stent according to an exemplary embodiment of the present invention is configured to include a stent body 2 and a spiral support part 4.

The stent body 2 includes a plurality of band parts, and is formed to configure a cylindrical stent form by these band parts.

The stent body 2 includes, for example, a first band part B1, a second band part B2, a third band part B3, and a fourth band part B4, and may be formed in a state in which the four band parts B1, B2, B3, and B4 are arranged to be connected to each other to configure a cylindrical form, as shown in FIG. 1.

The four band parts B1, B2, B3, and B4 may be provided in a form of elastic band bodies configuring cylindrical circumferential parts by supporting parts A of wires banded in a zigzag shape.

The spiral support part 4 is formed to perform a role of a support that may provide stable elastic restoring force to a circumference side of the stent body 2.

The spiral support part 4 is extended and formed to configure a spiral wound state along a circumference from one end portion of the stent body 2 in a length direction toward the other end portion of the stent body 2 in the length direction, as shown in FIG. 1.

Particularly, when the spiral support part 4 is extended in the spiral shape from one end portion of the stent body 2 in the length direction toward the other end portion of the stent body 2 in the length direction, the spiral support part 4 is extended and formed in a state in which it configures the supporting part A of any one point of each of the band parts B1, B2, B3, and B4 of the stent body 2.

That is, the spiral support part 4 has a structure in which support sections may be formed in a state in which the stent body 2 may configure one circumference.

In addition, when the spiral support part 4 is extended and formed in the spiral shape, it is preferable to form the spiral support part 4 to have spiral support sections wound along the circumference of the stent body 2 by at least one turn or more.

For example, the spiral support part 4 may be formed to have support sections wound along the circumference of the stent body 2 by approximately one turn , as shown in FIG. 1.

When the spiral support part 4 is formed to have a spiral section wound by a turn less than one turn, it is difficult to secure uniform elastic restoring force corresponding to the entire circumference of the stent body 2.

In addition, when the spiral support part 4 is extended in the spiral shape from one end portion of the stent body 2 in the length direction toward the other end portion of the stent body 2 in the length direction, it is formed along the circumference of the stent body 2 in a state in which a disconnected (or connected) portion or a bent portion is not present in the support sections.

According to the structure of the spiral support part 4 described above, the uniform elastic restoring force may be secured by a simple support structure integrated with the stent, as compared with, for example, a support formed by separately adding a wire onto a stent circumference although not illustrated in the drawings or support structures in which support sections are disconnected or bent.

Next, a method of manufacturing a stent according to an exemplary embodiment of the present invention will be described with reference to FIGS. 2 to 12.

FIG. 2 is a view showing processes of a method of manufacturing a stent. In FIG. 2, reference numeral S1 indicates forming a spiral support part, and reference numeral S2 indicates forming a stent body.

The forming (S1) of the spiral support part and the forming (S2) of the stent body are performed in a scheme of banding a wire W using a frame for manufacturing a stent, and the frame for manufacturing a stent has a structure in which a plurality of banding points are formed on a cylindrical outer surface thereof.

FIG. 3 is a development view showing an outer circumferential surface of the frame for manufacturing a stent in a developed state. The frame for manufacturing a stent may be configured in a frame structure in which a plurality of graduations are partitioned in an x direction corresponding to a circumference of the stent and a y direction corresponding to a length of the stent and pins are installed at appropriate points so that intersection points between these graduations may be used as banding points.

The forming (S1) of the spiral support part is performed in a scheme of banding the wire W in a spiral wound state from one end of the stent in the length direction toward the other end of the stent in the length direction.

In this case, the wound state of the wire W may be formed to have, for example, spiral support sections extended to be wound along the circumference of the stent by approximately one turn, as shown in FIG. 1.

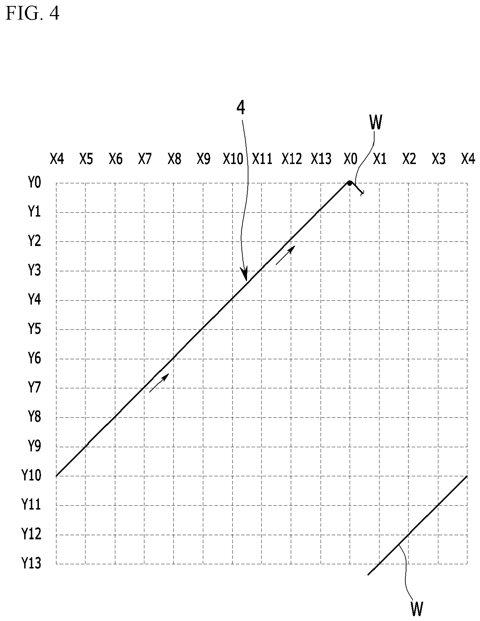

That is, in FIG. 4, the wire W is extended from a banding point (x1, y13) toward a banding point (x0, y0).

In this case, the spiral support part 4 extended in the spiral wound state from one end of the stent body in the length direction toward the other end of the stent body in the length direction may be formed.

Next, the forming (S2) of the stent body is performed.

The forming (S2) of the stent body is performed in a scheme of banding the wire W in a zigzag shape at an extended distal point of the spiral support part 4 so as to correspond to the wound state of the spiral support part 4 to sequentially form a plurality of band parts configuring a cylindrical shape.

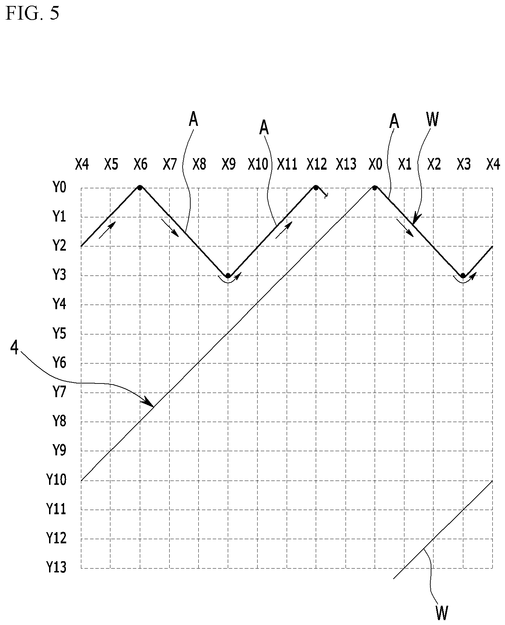

That is, in FIG. 5, the wire W is banded in an extended state having a zigzag shape so as to configure the circumference of the stent while passing through a path of x3y3, x6y0, x9y3, and x12y0 rightward from a banding point (x0,y0).

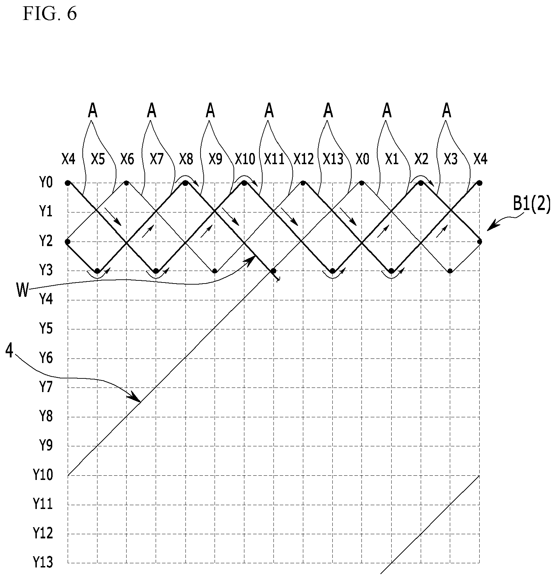

Then, in FIG. 6, the wire W is banded in an extended state having a zigzag shape while passing through paths of x1y3, x4y0, x7y3, and x10y0 and x13y3, x2y0, x5y3, x8y0, and x11y3 rightward from a banding point (x12y0).

In this case, a first band part B1 configuring a cylindrical shape corresponding to the circumference of the stent may be formed by supporting parts A of the wire W banded in the zigzag shape.

When forming the first band part B1 while banding the wire W in the zigzag shape as described above, the supporting parts A are in an intersection state in which any one thereof passes through the other thereof above or below the other thereof at intersection points between the supporting parts A. Such a banding scheme is also similarly applied to a case of forming a second band part B2, a third band part B3, and a fourth band part B4 to be described below.

Particularly, when the wire W is banded as described above, a section of a supporting part A between banding points (x11y3 and x0y0) as shown in FIG. 6 among supporting parts A configuring a circumference of the first band part B1 is formed by some of support sections of the spiral support part 4.

That is, the spiral support part 4 is formed to perform a support action in a state in which it configures any one A of the supporting parts A of the first band part B1.

Next, the wire W is again banded at a distal point of the first band part B1 to form a second band part B2 connected to the first band part B1.

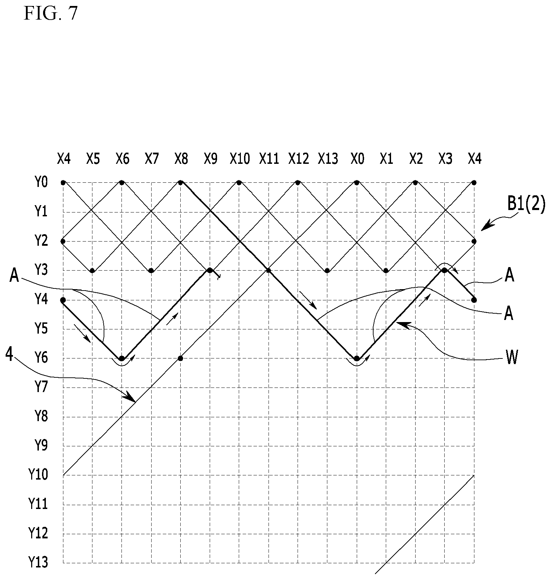

In FIG. 7, the wire W is banded in an extended state having a zigzag shape so as to configure the circumference of the stent while passing through a path of x0y6, x3y3, x6y6, and x9y3 rightward from a banding point (x11,y3).

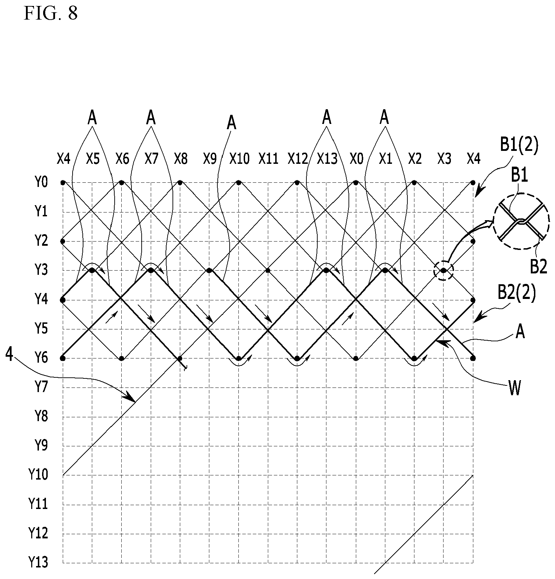

Then, in FIG. 8, the wire W is banded in an extended state having a zigzag shape while passing through paths of x12y6, x1y3, x4y6, and x7y3 and x10y6, x13y3, x2y6, x5y3, and x8y6 rightward from a banding point (x9y3).

In this case, a second band part B2 may be formed in a state in which it configures a cylindrical shape together with the first band part B1 while having a circumference formed by supporting parts A of the wire W banded in the zigzag shape.

Particularly, when the wire W is banded as described above, a section of a supporting part A between banding points (x8y6 and x11y3) (banding start points of the second band part) as shown in FIG. 8 among the supporting parts A configuring the circumference of the second band part B2 is formed by some of the support sections of the spiral support part 4.

That is, the spiral support part 4 is formed to perform a support action in a state in which it configures any one A of the supporting parts A of the second band part B2 as well as the first band part B1.

Next, the wire W is again banded at a distal point of the second band part B2 to form a third band part B3 connected to the second band part B2.

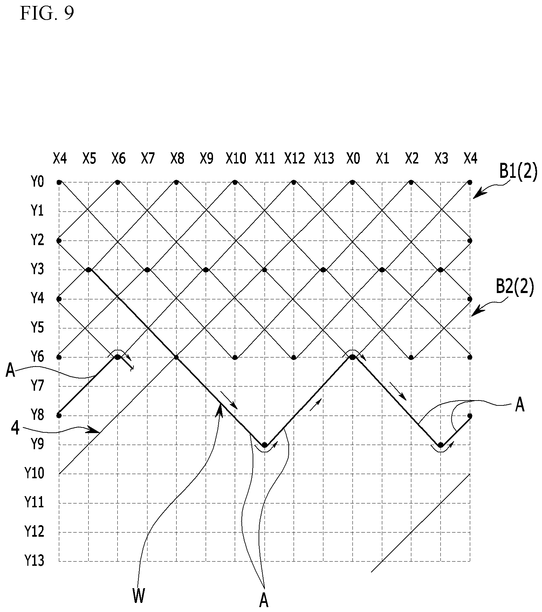

In FIG. 9, the wire W is banded in an extended state having a zigzag shape so as to configure the circumference of the stent while passing through a path of x11y9, x0y6, x3y9, and x6y6 rightward from a banding point (x8,y6).

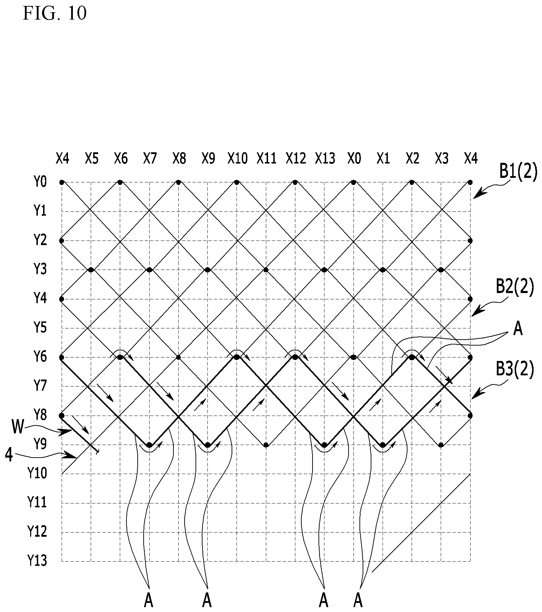

Then, in FIG. 10, the wire W is banded in an extended state having a zigzag shape while passing through paths of x9y9, x12y6, x1y9, and x4y6 and x7y9, x10y6, x13y9, x2y6, and x5y9 rightward from a banding point (x6y6).

In this case, a third band part B3 may be formed in a state in which it configures a cylindrical shape together with the first band part B1 and the second band part B2 while having a circumference formed by supporting parts A of the wire W banded in the zigzag shape.

Particularly, when the wire W is banded as described above, a section of a supporting part A between banding points (x5y9 and x8y6) (banding start points of the third band part) as shown in FIG. 10 among the supporting parts A configuring the circumference of the third band part B3 is formed by some of the support sections of the spiral support part 4.

That is, the spiral support part 4 is formed to perform a support action in a state in which it configures any one A of the supporting parts A of the third band part B3 as well as the first band part B1 and the second band part B2.

Next, the wire W is again banded at a distal point of the third band part B3 to form a fourth band part B4 connected to the third band part B3.

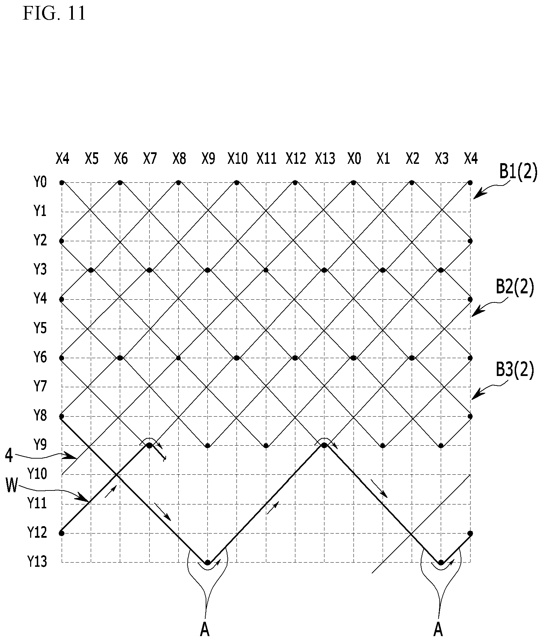

In FIG. 11, the wire W is banded in an extended state having a zigzag shape so as to configure the circumference of the stent while passing through a path of x9y13, x13y9, x3y13, and x7y9 rightward from a banding point (x5,y9).

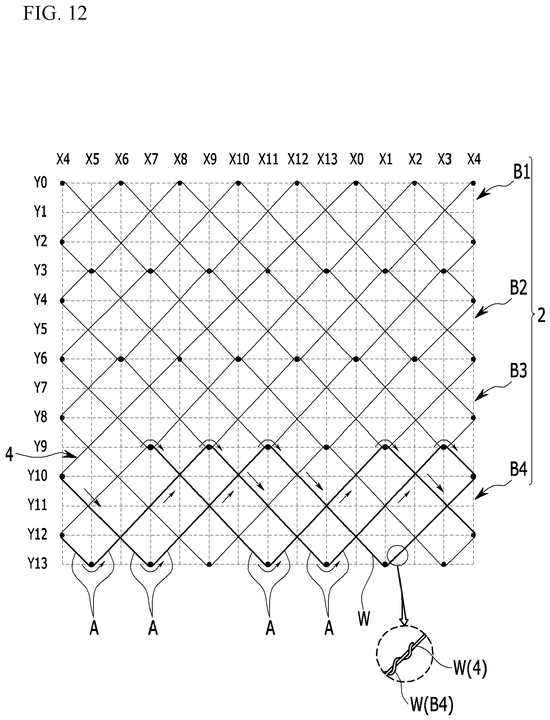

Then, in FIG. 12, the wire W is banded in an extended state having a zigzag shape while passing through paths of x11 y13, x1y9, x5y13, and x9y9 and x13y13, x3y9, x7y13, x11 y9, and x1 y13 (banding start points of the spiral support part) rightward from a banding point (x7y9).

In addition, a distal end of the wire W at a banding point (x1y13) is connected and finished to a start end portion of the spiral support part 4 by a general method.

In this case, a fourth band part B4 may be formed in a state in which it configures a cylindrical shape together with the first band part B1, the second band part B2, and the third band part B3 while having a circumference formed by supporting parts A of the wire W banded in the zigzag shape.

Particularly, when the wire W is banded as described above, a section of a supporting part A between banding points (x1y13 and x5y9) (banding start points of the fourth band part) as shown in FIG. 12 among the supporting parts A configuring the circumference of the fourth band part B4 is formed by some of the support sections of the spiral support part 4.

That is, the spiral support part 4 is formed to perform a support action in a state in which it configures any one A of the supporting parts A of the fourth band part B4 as well as the first band part B1, the second band part B2, and the third band part B3.

In addition, when the band parts B1, B2, B3, and B4 as described above are sequentially formed to be connected to each other by banding the wire W, the wire W is banded so that an end portion (for example, a valley portion) of the supporting part A of the first band part B1 and an end portion (for example, a peak portion) of the supporting part A of the second band part B2 are entangled and connected to each other, for example, as shown in FIG. 7, at connection points between the band parts B1, B2, B3, and B4.

Therefore, in the forming (S2) of the stent body, the wire W may be banded to form the stent body 2 in a connection state in which the plurality of band parts B1, B2, B3, and B4 configure the cylindrical shape.

Therefore, in the stent according to an exemplary embodiment of the present invention, a stent structure in which the stent body 2 formed to configure the cylindrical shape by the banding of the wire W and the spiral support part 4 disposed along the circumference of the stent body 2 are formed integrally with each other may be provided.

Particularly, the spiral support part 4 is formed to have the support sections continuously extended and formed to configure a spiral shape along the circumference from one end of the stent in the length direction toward the other end of the stent in the length direction in a state in which it configures any one supporting part A forming the circumferences of the respective band parts B1, B2, B3, and B4 of the stent body 2.

In the structure of the spiral support part 4 described above, the spiral support part 4 may be formed along the circumference of the stent in a state in which it configures one circumference of the stent. Therefore, the structure of the spiral support part 4 is simpler than support structures in which the wire is additionally wound around the circumference of the stent, such that support sections are formed in a state in which they irregularly protrude on inner and outer surfaces of the stent or support sections are discontinuously extended and formed, thereby making it possible to further improve stenting stability and provide more uniform elastic restoring force to the circumference of the stent.

## Claims

1. A method of manufacturing a stent, comprising:
a) forming a spiral support part by banding a wire so as to have a spiral wound state from one side toward the other side; and
b) banding the wire in a state in which the spiral support part configures a portion of a circumference to form a plurality of band parts and forming a stent body so that the band parts configure a connection state having a cylindrical shape.

2. The method of manufacturing a stent of claim 1, wherein:
in the step a),
the wire is banded to have a spiral wound state configuring a circumference of the stent body from one end portion point of the stent body in a length direction toward the other end portion point of the stent body in the length direction.

3. The method of manufacturing a stent of claim 2, wherein:
in the step a),
the wire is banded to configure a wound state in which it is wound around the circumference of the stent body by one turn or more.

4. The method of manufacturing a stent of claim 2, wherein:
in the step a),
the wire is banded to have spiral support sections extended and formed integrally with each other from one end portion point of the stent body in the length direction toward the other end portion point of the stent body in the length direction.

5. The method of manufacturing a stent of claim 1, wherein:
in the step b),
the wire is banded so as to have supporting parts arranged in a connection state configuring a zigzag shape to form the band parts in a state in which the supporting parts configure the circumference of the stent together with the spiral support part.

6. The method of manufacturing a stent of claim 5, wherein:
in the step b),
a wire banding process is performed in a state in which a supporting part of any one point among the supporting parts forming circumferences of the band parts is formed by some of support sections of the spiral support part.

7. The method of manufacturing a stent of claim 5, wherein:
in the step b),
a banding process of banding the wire in the zigzag shape to form any one band part and banding the wire in the zigzag shape to have an entanglement state in which it configures a cylindrical shape together with any one band part to extend and form another band part is performed.
